# EUROPÄISCHE PATENTSCHRIFT

(11) **EP 1 206 226 B1**
(45) Veröffentlichungstag und Bekanntmachung des Hinweises auf die Patenterteilung: **10.11.2004**
(21) Anmeldenummer: 00945802.7
(22) Anmeldetag: 21.06.2000
(51) Int. Cl.: A61F 2/44

(54) **IMPLANTAT AUS KNOCHENMATERIAL**
IMPLANT FROM OSSEOUS MATERIAL
IMPLANT EN MATIERE OSSEUSE

(30) Priorität: 28.07.1999 DE 29913200 U
(43) Veröffentlichungstag der Anmeldung: 22.05.2002
(73) Patentinhaber: Tutogen Medical GmbH, 91077 Neunkirchen am Brand (DE)
(72) Erfinder: BOULARD, Erik, F-31790 Saint-Sauveur (FR); KALAS, Rolf-Dieter, D-34320 Söhrewald (DE); KOSCHATZKY, Karl, D-91058 Erlangen (DE); KRÜGER, Manfred, D-61389 Arnoldshain (DE)
(74) Vertreter: Manitz, Finsterwald & Partner GbR
(86) Internationale Anmeldenummer: PCT/EP2000/005764
(87) Internationale Veröffentlichungsnummer: WO 2001/008611

(56) Entgegenhaltungen:
- WO-A-98/42269
- WO-A-99/09914
- DE-A- 4 409 836
- US-A- 4 950 296

## Beschreibung

Die vorliegende Erfindung betrifft ein Implantat zur Verbindung von Knochen und insbesondere ein Wirbelsäulenimplantat zur Fusion von Wirbelknochen, das zwischen zwei zu fusionierende Wirbelknochen eingesetzt wird.

Durch die Degeneration der Bandscheibe, insbesondere des Bandscheibenkems (Nucleus pulposus) kommt es zu einem Höhenverlust im betroffenen Bandscheibenfach, der mit einer Lockerung des Bandscheibenringes (Annulus fibrosus) und der Bänder verbunden ist. Dadurch wird die Wirbelsäule an dieser Stelle instabil. Die Folge ist eine horizontale Verschiebbarkeit der Wirbelkörper gegeneinander (Spondylolisthese), die zu Beeinträchtigungen der Nervenwurzeln in diesem Bereich und oder des Rükkenmarks mit daraus resultierenden Schmerzen führt. Ähnliche Symptome können nach chemisch-enzymatischer oder physikalischer (Laser) Auflösung des Bandscheibenkerns (Nucleolyse) zur Behandlung eines Bandscheibenvorfalls auftreten (Postnukleolyse-Syndrom).

Das Prinzip zur Behandlung dieser Symptome besteht in der operativen Ausräumung des Bandscheibenkerns und dem Einlegen bzw. Einschieben - im Bereich der Halswirbelsäule - eines oder - im Bereich der Lendenwirbelsäule - zweier ausreichend stabiler Körper, um die normale Höhe des Bandscheibenfaches wiederherzustellen. Gleichzeitig muß die horizontale Verschiebbarkeit verhindert werden. Dies geschieht entweder durch das Implantat selbst oder durch zusätzliche Metallimplantate (instrumentierte Fusion). Diese Implantate unterliegen insbesondere in der Lendenwirbelsäule erheblichen Kräften, die zum Bruch des Metallimplantats führen können. Es wird daher angestrebt, daß das Zwischenwirbelinterponat möglichst rasch und solide mit den angrenzenden Wirbelkörpern verwächst bzw. fusioniert.

Zur Behandlung von Patienten mit spinalem Trauma oder degenerativen Leiden an der Wirbelsäule werden im wesentlichen zwei Techniken angewendet.
1. Entfernen des Bandscheibenkerns und des Knorpels an den Endplatten, Aufdehnen des Zwischenwirbelraumes auf normale Weite und Einschieben eines planparallelen oder horizontal leicht keilförmigen Blocks (Smith-Robinson-Technik).
2. Aufdehnen des Bandscheibenfaches auf normale Höhe, Bohren eines zylindrischen Loches, das beide Wirbelkörper erfaßt, und Einschieben eines zylindrischen Dübels (Cloward-Technik). Der Dübel kann dabei entweder ein glatter Zylinder sein oder die Form einer Maschinenschraube haben.

Eine bekannte Möglichkeit zur Fusion zweier Wirbel besteht demnach im Einschub eines geeignet geformten Zylinders oder Dübels in ein vorbereitetes, die beiden zu fusionierenden Wirbel erreichende Kavität. Das dazu benötigte Material wird dem Patienten vorher beispielsweise aus dem Bekkenkamm entnommen. Aus dem so gewonnenen autogenen, d.h. vom gleichen Patienten stammenden Knochenmaterial wird ein Implantat angefertigt und anschließend dem Patienten in den Wirbelzwischenraum zwischen den zu fusionierenden Wirbeln eingesetzt (Autograft). Alternativ können auch Fusionsdübel verwendet werden, deren Material aus allogenem, kortikalem oder kortiko-spongiösem Knochenmaterial eines fremden Spenders gewonnen wird (Allograft) oder die aus spongiösem oder kortiko-spongiösem, bovinem Material (Xenograft) hergestellt sind.

Die zuerst genannte Methode hat den Nachteil, daß sie eine hohe Rate an kollabierenden Knochen mit unzureichender biomechanischer Stabilität mit sich bringt und daß die erforderliche Zweitoperation zur Gewinnung von zusätzlichem Knochenmaterial dem Patienten Schmerzen zufügt, die größer sein können als die Schmerzen, die operativ beseitigt werden sollen.

Weiterhin ist es bekannt, die für die betreffende Behandlungsmethode verwendeten Fusionsdübel mit einem Außengewinde zu versehen, um damit einen besseren Halt zwischen den zu fusionierenden Wirbeln zu erzielen. Bei der Implantation kann eine natürlich vorhandene Öffnung im Dübel, wie ein medullärer Kanal, vertikal zur Einschubrichtung des Implantats in die Wirbel oder eine gebohrte Öffnung im Knochendübel derart genutzt werden, daß diese Öffnung mit spongiösem Knochen gefüllt wird, das vom Patienten selbst oder auch von fremden Spendern stammen kann. Durch das schnelle Remodellierungsverhalten des eingefüllten, spongiösen Knochens wird mit der Einheilung eine verwachsene, knöcherne Verbindung der zu fusionierenden Wirbel erzeugt.

Die bekannten Fusionsdübel haben jedoch den Nachteil, daß zum sicheren Sitz der Dübel die Endplatten der zu fusionierenden Wirbelkörper ausgebohrt werden müssen. Dadurch entsteht die Gefahr, daß der Dübel in den Wirbelkörper einsinkt, was zum Höhenverlust und damit zu erneuter Schädigung der Nervenwurzeln führen kann.

Das Versagen ist einerseits durch den großen Durchmesser der eingesetzten Fusionsdübel bedingt, der üblicherweise 12 bis 16 mm beträgt, und andererseits durch die Größe des für das Implantat benötigten Bohrlochs, wobei die für das Setzen der Bohrung und das Einbringen des Implantats notwendige seitliche Verlagerung des Rückenmarks erhebliche Zugkräfte auf die Nervenwurzeln auslöst. Darüber hinaus ist das Einbringen des erforderlichen Bohrlochs und die damit verbundene Vergrößerung des Raumes zwischen den Wirbelfortsätzen für den Heilungsprozeß ungünstig, da dabei gesunder Knochen aus den Wirbeln ausgeräumt und eine Schwächung der Wirbel herbeigeführt wird. Weiterhin nachteilig ist, daß die zylinderförmigen Fusionskörper keine Krümmung der Wirbelsäule nach vorne (Lordose) erlauben.

Eine andere bekannte Möglichkeit zur Fusion zweier Wirbel besteht in dem Einschub von Implantaten mit sogenannten Käfigen bzw. Cages aus Metall oder Kunststoff. Diese Käfige besitzen einen Hohlraum, der mit spongiösem, autogenen Knochenmaterial oder auch mit allogenem Knochenmaterial gefüllt werden kann. Die Käfige haben üblicherweise eine zylindrische Form, um sie in ein in der Wirbelsäule vorbereitetes Bohrloch einzuschieben. Daneben sind auch Käfige mit flachen, einem Brikett ähnlichen Formen bekannt, die in den Wirbelzwischenraum eingeschoben werden, ohne daß dazu in der Wirbelsäule ein Bohrloch vorbereitet werden muß. Bei diesen Käfigen mit flacher Form wird der durch Ausräumen der Zwischenwirbelkörper (Bandscheiben) entstandene Raum zwischen den zu fusionierenden Wirbeln zum Einschub genutzt.

Bei den bekannten Käfigen für Implantate wirkt sich jedoch die Verwendung von Metallen oder Kunststoffen nachteilig aus, da diese nicht biologischen Materialien als Fremdkörper im Körper des Patienten verbleiben und damit Abstoßungsreaktionen des Körpers gegen das Implantat hervorrufen können. Aufgrund der Tatsache, daß eine Einheilung dieser Implantate in den Knochen mit solchen nicht biologischen Materialien nicht möglich ist, besteht eine dauernde Schwächung im Fusionsbereich zwischen den betreffenden Wirbeln. Darüber hinaus wirkt sich die Rigidität der Metallkäfige nachteilig aus, da sie höher ist als diejenige des Wirbelknochens und somit eine ständige mechanische Beanspruchung der Wirbelsäule verursacht, die zum Einsinken der Käfige in die Wirbelkörperendplatten führen kann.

Ein Wirbelsäulenimplantat nach dem Oberbegriff des Anspruchs 1 ist aus der WO-A-99/09914 bekannt.

Die Aufgabe der vorliegenden Erfindung besteht deshalb darin, ein Implantat zur Fusion von Knochen zu schaffen, das die oben genannten Nachteile beseitigt. Die Aufgabe der Erfindung besteht insbesondere darin, ein stabiles Implantat zur Fusion zweier Wirbel zu schaffen, das den korrekten Abstand zwischen den Wirbelkörpern und die Stabilität der Wirbelsäule wiederherstellt, eine korrekte Statik der Wirbelsäule gewährleistet, den korrekten Nervenverlauf in der Wirbelsäule sicherstellt und dabei keine Körperabwehrreaktionen hervorruft.

Diese Aufgabe wird gelöst durch ein Wirbelsäulenimplantat bestehend aus einem Rahmen aus kortikalem Knochenmaterial, der mindestens einen durchgehenden Hohlraum bildet, der mindestens zwei gegenüberliegende Öffnungen aufweist und zumindest teilweise mit spongiösem und/oder osteoinduktivem Knochenmaterial gefüllt ist, wobei der Rahmen im wesentlichen quaderförmig ist und zumindest auf einer Seite ballige Randabschnitte aufweist, wobei an dem Rahmen Strukturierungen vorgesehen sind, um das Wirbelsäulenimplantat zwischen benachbarten Wirbeln zu fixieren, und wobei der Rahmen mindestens eine Aufnahmeöffnung für ein Applikationswerkzeug aufweist.

Ein besonderer Vorteil des Wirbelsäulenimplantats gemäß der Erfindung ist durch das verwendete Material gegeben, das aufgrund seines biologischen Ursprungs und einer speziellen Konservierung keinen Fremdkörper darstellt. Dadurch trägt das aus Knochenmaterial hergestellte Implantat in seiner Gesamtheit, also sowohl der Rahmen aus kortikalem Knochenmaterial als auch die Füllung aus spongiösem Knochenmaterial, zur Fusion der Knochen bei, indem es sich während der Einheilung in körpereigenes Knochengewebe umwandelt. Alternativ können anstatt spongiösem Knochenmaterial auch osteoinduktive Knochenmaterialien in den Hohlraum des Implantats eingebracht werden, welche die Knochenfusion über ihr osteoinduktives Potential beschleunigen können.

Die Fusionswirkung des erfindungsgemäßen Implantats wird besonders durch die Gestaltung des Rahmens begünstigt, der auf den Seiten, die im applizierten Zustand den zu fusionierenden Wirbelkörpern gegenüberliegen jeweils mindestens eine Öffnung aufweist. Auf diese Weise kommt das spongiöse Knochenmaterial im Hohlraum des Rahmens mit den Knochenoberflächen der Wirbelkörper in direkten Kontakt, um mit diesen eine knöcherne Verbindung auszubilden und sie dadurch miteinander zu verbinden bzw. zu fusionieren. Durch die besondere Gestaltung des Rahmens des erfindungsgemäßen Wirbelsäulenimplantats ist dessen Einsatz sowohl im lumbalen als auch im cervikalen Wirbelzwischenraum möglich, ohne daß dabei eine Bohrung in den zu fusionierenden Wirbeln vorbereitet werden muß und damit ohne eine signifikante Schwächung des gesunden und zu erhaltenden Wirbelknochens.

Ein weiterer Vorteil ergibt sich daraus, daß der Rahmen zumindest auf einer Seite ballige Randabschnitte aufweist. Aufgrund dieser Formgebung ist das erfindungsgemäße Wirbelsäulenimplantat zwar eine höhere Disktraktion, erreicht aber durch die Ballung einen besseren flächenhaften Kontakt zur leicht invaginierten, distalen Endplatte des Wirbelknochens. Wenn diese Seite mit dem balligen Randabschnitt einer zu fusionierenden Knochenoberfläche zugewandt ist, kann auch eine Krümmung der Wirbelsäule nach vorne (Lordose) erreicht werden.

Nach einem weiteren Aspekt der vorliegenden Erfindung kann auf mindestens einer Seite des Rahmens eine oder mehrere Aufnahmeöffnungen für ein Werkzeug vorgesehen sein, das beispielsweise als Gabel ausgebildet ist, mit dessen Hilfe das Wirbelsäulenimplantat in den Wirbelzwischenraum zwischen die zu fusionierenden Wirbel eingebracht werden kann. Dadurch wird die Applikation des Wirbelsäulenimplantats erleichtert.

In der Beschreibung, der Zeichnung und den Unteransprüchen sind weitere vorteilhafte Ausgestaltungen des Wirbelsäulenimplantats gemäß der vorliegenden Erfindung angegeben.

Das Material des Rahmens besteht erfindungsgemäß aus prozessiertem, konservierten und sterilen Knochenmaterial humanen Ursprungs, sogenanntem Allograft, oder aus prozessiertem, konserviertem und sterilem Knochenmaterial tierischen Ursprungs, sogenanntem Xenograft. Der Rahmen kann aus kortikalem Knochenmaterial gefertigt werden, beispielsweise aus Humerus, Femur, Tibia, oder anderen Knochen sowohl von verstorbenen Menschen als auch von Tieren, insbesondere aus Knochenmaterial vom Rind hergestellt werden.

Bei einer besonders vorteilhaften Ausgestaltung des erfindungsgemäßen Wirbelsäulenimplantats weist der im wesentlichen quaderförmige Rahmen abgerundete Kanten auf. Eine solche Gestaltung des Rahmens erleichtert die Applikation des Wirbelsäulenimplantats zwischen den zu fusionierenden Wirbelkörpern, indem die Abrundungen ein Verkanten des Implantats während der Applikation vermeiden.

Vorzugsweise weist der Rahmen des erfindungsgemäßen Wirbelsäulenimplantats zumindest auf beiden Seiten, die im applizierten Zustand den zu fusionierenden Wirbelknochen gegenüberliegen, einen umlaufenden Randbereich auf, der die jeweilige Öffnung des durchgehenden Hohlraums umgibt. Dadurch ist gewährleistet, daß der Hohlraum leicht zugänglich ist, um ihn mit spongiösem Knochenmaterial zu füllen, und daß die spongiöse Füllung mit den zu fusionierenden Wirbeln nach oben (cranial) sowie nach unten (caudal) in direkten und flächigen Kontakt kommt.

Gemäß einer bevorzugten Ausführungsform der vorliegenden Erfindung ist der Rahmen des Wirbelsäulenimplantats derart gestaltet, daß dabei die gegebene Form der Wirbelkörper oberhalb bzw. unterhalb des eingeschobenen Wirbelsäulenimplantats je nach Einsatzort, beispielsweise bei lumbaler oder cervikaler Anwendung, berücksichtigt ist. Das heißt, daß der Rahmen des Wirbelsäulenimplantats so geformt ist, daß er durch eine entsprechende Wölbung der balligen Randabschnitte an die gegebene Form der Wirbelkörper angepaßt ist. Dadurch wird ein flächiger Kontakt erzielt, der die Einheilung des Implantats begünstigt und die Stabilität der behandelten Wirbelsäule verbessert.

Bei einer weiteren bevorzugten Ausführungsform des erfindungsgemäßen Wirbelsäulenimplantats weist der Rahmen auf zwei gegenüberliegenden Seiten ballige Randabschnitte auf, um das Implantat an die vorgegebene natürliche Wölbung der Wirbelendplatten, mit denen das Implantat in Kontakt kommt, anzupassen. Die sich gegenüberliegenden balligen Randabschnitte können ferner sowohl im Randbereich um die obere Öffnung als auch im Randbereich um die untere Öffnung des Rahmens vorgesehen sein. Darüber hinaus kann auch die Füllung aus spongiösem Knochenmaterial gewölbt bzw. ballig ausgebildet sein, um sie an die gegebene Form der Wirbelendplatten anzupassen.

Besonders vorteilhaft ist es, wenn die Randbereiche des Wirbelsäulenimplantats gemäß der Erfindung auf zwei gegenüberliegenden Seiten des Rahmens jeweils Strukturierungen aufweisen, die in Form von Kerbungen, Riffelungen oder Zinnen ausgebildet sind. Die Einrichtung solcher Strukturierungen an den Kontaktseiten des Rahmens mit dem Knochen haben die vorteilhafte Wirkung, daß Sitz und Verankerung des Wirbelsäulenimplantats an den Wirbelkörpern verbessert werden.

Das Implantat ist in seiner Größe vorzugsweise an den nach Ausräumung des Wirbelzwischenkörpers vorhandenen Wirbelzwischenraum zwischen benachbarten Wirbeln angepaßt. Die Applikation des erfindungsgemäßen Wirbelsäulenimplantats erfolgt nach Ausräumen der Bandscheibe mit anschließender Freilegung der darunter- und darüberliegenden Wirbelkörper, ohne daß dabei gesundes Knochenmaterial beschädigt werden muß.

Dies bringt die Vorteile mit sich, daß der bestehende Wirbelzwischenraum nicht vergrößert wird und die tragenden Strukturen erhalten bleiben.

Nach einem weiteren Aspekt der vorliegenden Erfindung sind die Aufnahmeöffnungen auf einer oder mehreren Seiten des Rahmens als Bohrloch mit einem Gewinde von vorzugsweise 3 mm Tiefe ausgeführt. In diese Gewindebohrungen können dann mit passendem Gewinde versehene Applikationswerkzeuge eingeschraubt werden, um das Implantat zwischen die zu fusionierenden Wirbel einzubringen.

Als Material wird für das Wirbelsäulenimplantat gemäß der vorliegenden Erfindung ein geeignetes allogenes oder xenogenes Knochenmaterial derart prozessiert, daß es konserviert, lagerfähig sowie steril ist und bestimmungsgemäß eingesetzt werden kann. Die Konservierung des Knochenmaterials kann beispielsweise mittels Gefriertrocknung erfolgen. Das spongiöse Knochenmaterial ist vorzugsweise durch Lösungsmitteldehydratisieren von nativem Knochenmaterial mittels eines organischen, mit Wasser mischbaren Lösungsmittels, z.B. Methanol, Äthanol, Propanol, Isopropanol, Aceton, Methyl-Äthylketon oder Gemischen dieser Lösungsmittel, erzeugt. Die Konservierung und Sterilisation des Knochenmaterials nach diesem Verfahren ist auch Gegenstand des Patents DE 29 06 650, dessen Inhalt durch diese Bezugnahme in die Offenbarung der vorliegenden Anmeldung aufgenommen wird.

Dieses Verfahren dient der Herstellung von Transplantatkonserven und ermöglicht eine Dehydratisierung und Freilegung bis in den Feinbau der Fibrille des Knochenmaterials, so daß das prozessierte Knochenmaterial im histologischen Bild eine dem natürlichen Knochen sehr ähnliche morphologische Struktur aufweist und somit die gewünschten Eigenschaften des Knochenmaterials erhalten bleiben. Dieses Verfahren der Lösungsmitteldehydratisierung hat außerdem den Vorteil, daß im Vergleich zur Gefriertrocknung ein wesentlich geringerer apparativer Aufwand erforderlich ist.

Ferner kann das spongiöse Knochenmaterial auch durch Lösungsmitteldehydratisierung von Knochenmaterial mit anschließender terminaler Sterilisation, insbesondere durch Bestrahlung mit Gamma-Strahlen erzeugt werden. Alternativ kann das spongiöse Knochenmaterial durch aseptische Prozessierung von Knochenmaterial ohne terminale Sterilisation erzeugt werden.

Anschließend wird die vorliegende Erfindung rein exemplarisch anhand von Ausführungsbeispielen eines erfindungsgemäßen Wirbelsäulenimplantats unter Bezugnahme auf die beigefügten Zeichnungen beschrieben. Es zeigen:
- Fig. 1 und 2: jeweils eine perspektivische Ansicht einer Ausführungsform eines Wirbelsäulenimplantats gemäß der vorliegenden Erfindung; und
- Fig. 3: eine perspektivische Ansicht einer weiteren Ausführungsform eines Wirbelsäulenimplantats gemäß der vorliegenden Erfindung.

In den Figuren bezeichnen die gleichen Bezugszeichen jeweils die gleichen Komponenten der dargestellten Ausführungsformen. Die dargestellten Ausführungsbeispiele sind sowohl für die cervikale als auch für die lumbale Fusion von Wirbelknochen geeignet.

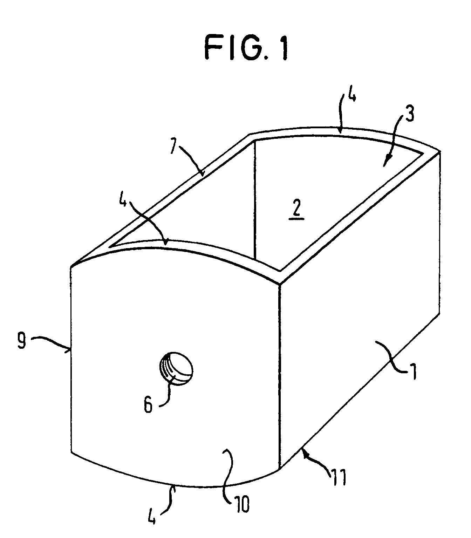

Das in Fig. 1 dargestellte Ausführungsbeispiel eines erfindungsgemäßen Wirbelsäulenimplantats umfaßt einen Rahmen 1, der beispielsweise aus kortikalem, diaphysärem Knochenmaterial z.B. humanen Ursprungs besteht. Dieser Rahmen bildet einen durchgehenden Hohlraum 2, der jeweils über Öffnungen 3 und 11 nach oben und nach unten geöffnet ist, wobei die untere Öffnung 11 in der Zeichnung nur mittelbar zu erkennen ist. Der Rahmen 1 ist im wesentlichen quaderförmig und weist an seiner oberen Umrandung, die im applizierten Zustand mit der Knochenoberfläche des zu fusionierenden Wirbelkörpers (nicht gezeigt) in Kontakt tritt, einen umlaufenden Randbereich 7 auf. Im dargestellten Fall umgibt der Randbereich 7 die Öffnung 3 des durchgehenden Hohlraums 2 vollständig und weist an den beiden sich gegenüberliegenden Breitseiten des Rahmens 1 jeweils ballige Randabschnitte 4 auf. Die untere Öffnung 11 des Rahmens 1 ist ebenfalls von einer Umrandung umgeben, deren Randbereich in gleicher Weise ausgestaltete ballige Randabschnitte aufweist.

Grundsätzlich ist der Rahmen 1 des Wirbelsäulenimplantats in seiner Größe an den vorgegebenen Platz, an dem das Implantat eingesetzt werden soll, angepaßt. Die Außenabmessungen eines solchen Wirbelsäulenimplantats können je nach Einsatzort bei cervikaler oder lumbaler Anwendung beispielsweise folgendermaßen sein: Länge 15 bis 23 mm, Breite 8 bis 13 mm, Höhe 5 bis 13 mm. Die Innenabmessungen des Rahmens 1 und damit die Abmessungen des Hohlraums 2 eines solchen Wirbelsäulenimplantats können beispielsweise folgendermaßen sein: Länge 11 bis 19 mm, Breite 4 bis 9 mm, Höhe 5 bis 13 mm. Die balligen Randabschnitte 4 an der Breitseite des Rahmens 1 sind je nach Einsatzort an die in der Zeichnung nicht dargestellte Wirbelkörperendplatte mit einem mittigen Maximum von ca. 1,0 bis 1,5 mm Höhe angepaßt.

Da der Rahmen 1 im wesentlichen quaderförmig ist, weist er Kanten 9 auf. Die in den Figuren vertikal verlaufenden Kanten 9, aber auch die übrigen Kanten, können abgerundet sein, um das Einbringen des Implantats zu erleichtern. Der Rahmen 1 weist ferner auf einer Seite 10 eine vorzugsweise mittig in der Seitenfläche 10 angeordnete Aufnahmeöffnung 6 auf, in die ein Applikationswerkzeug eingesetzt werden kann.

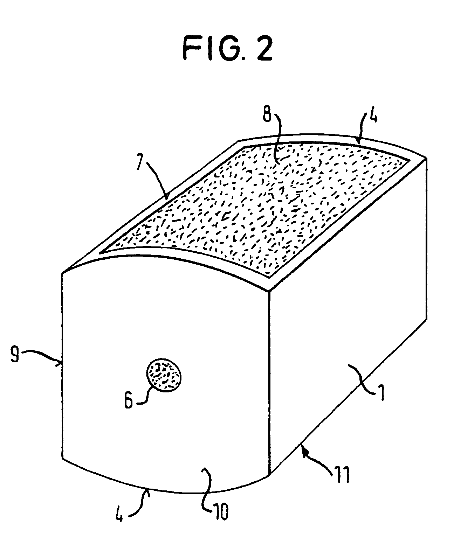

In Fig. 2 ist das Wirbelsäulenimplantat von Fig. 1 dargestellt, wobei der durch den Rahmen 1 gebildete durchgehende Hohlraum 2 mit spongiösem Knochenmaterial 8 gefüllt ist. Das spongiösem Knochenmaterial 8 kann humanen oder tierischen, insbesondere bovinen Ursprungs sein, und hat vorzugsweise osteoinduktive Eigenschaften, um den Einheilungsprozeß zu begünstigen. Die Füllung des Hohlraums 2 mit spongiösem und/oder osteoinduktivem Knochenmaterial erfolgt vorzugsweise derart, daß die Füllung 8 bei glatter, strukturfreier Ausführung des Randbereichs 7 des Rahmens 1 diesen über den gesamten Randbereich 7 auch in den balligen Randabschnitten 4 vorzugsweise um 0,5 mm überragt. Bei strukturierter Ausführung des Randbereichs 7 überragt die Füllung 8 den gesamten Randbereich 7 vorzugsweise bis zu der Höhe der freien Enden der Strukturierungen 5. Aufgrund des im Vergleich zum Füllmaterial 8 härteren Knochenmaterials des Randbereichs 7 und der darin geformten Strukturierungen wird auf diese Weise ein guter Kontakt der Spongiosa 8 des Wirbelsäulenimplantats im applizierten Zustand mit der Knochenoberfläche des zu fusionierenden Wirbelkörpers gewährleistet.

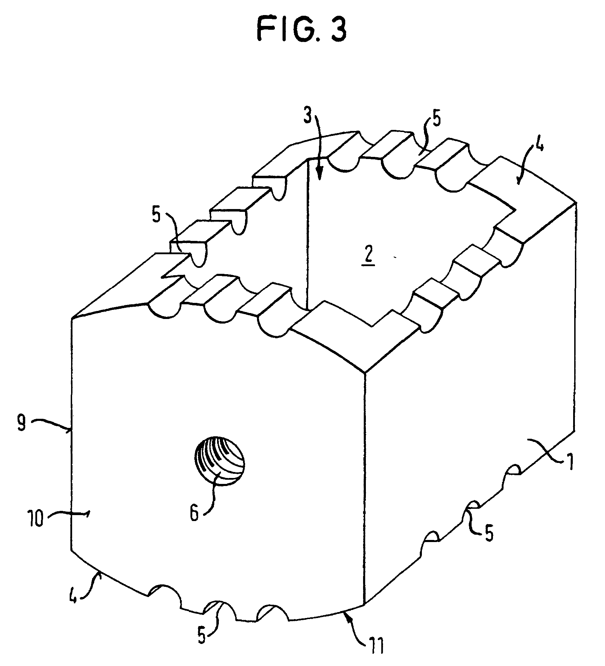

Fig. 3 zeigt eine weitere Ausführungsform der vorliegenden Erfindung, bei der an dem Rahmen 1 des Wirbelsäulenimplantats Strukturierungen 5 vorgesehen sind, um das Wirbelsäulenimplantat zwischen benachbarten Wirbeln zu fixieren. Diese Strukturierungen 5 sind vorzugsweise in den Randabschnitten 4 der die Öffnungen 3 und 11 umgebenden Randbereiche 7 vorgesehen, die im applizierten Zustand des Implantats mit der Knochenoberfläche eines zu fusionierenden Wirbelkörpers in Kontakt steht. Eine besonders gute Fixierung des Wirbelsäulenimplantats läßt sich erzielen, wenn die Strukturierungen 5 über den gesamten umlaufenden Randbereich 7 sowohl um die obere Öffnung 3 als auch um die untere Öffnung 11 des im wesentlichen quaderförmigen Rahmens 1 verteilt sind. Es können ferner alle mit den Wirbelkörperendplatten in Kontakt tretenden Berührungsflächen des Rahmens 1 strukturiert ausgeführt sein.

Die Strukturierungen 5 können beispielsweise in Form von Kerbungen, Riffelungen oder Zinnen ausgebildet sein. Vorzugsweise sind die Strukturierungen 5, wie bei der in Fig. 3 dargestellten Ausführungsform, auf jeder Seite des Randbereichs 7 des Rahmens 1 vorgesehen und zinnenförmig ausgestaltet. Dazu sind in der Mitte jeder Seite des Randbereichs 7 jeweils drei Vertiefungen mit dazwischenliegenden Zinnen ausgebildet, die so ausgerichtet sind, daß sie sich in der anliegenden Knochenoberfläche verzahnen, wenn das Implantat bestimmungsgemäß zwischen den zu fusionierenden Wirbeln eingesetzt ist.

Bei dem in Fig. 3 dargestellten Ausführungsbeispiel ist zu erkennen, daß die Aufnahmeöffnung 6 als Bohrloch in dem Rahmen 1 mit einem Gewinde von vorzugsweise 3 mm Tiefe ausgeführt sein kann, in das ein mit passendem Gewinde versehenes Applikationswerkzeug eingeschraubt werden kann. Wie bei dem dargestellten Ausführungsbeispiel, ist die Aufnahmeöffnung 6 vorzugsweise in der Mitte der Seitenfläche 10 des Rahmens 1 angeordnet. Die Applikation des erfindungsgemäßen Wirbelsäulenimplantats kann zusätzlich erleichtert werden, wenn Aufnahmeöffnungen 6 für ein Applikationswerkzeug auf gegenüberliegenden Seiten 10 des Rahmens 1 vorgesehen sind, um das Implantat beispielsweise mit einem zangenartigen Werkzeug ergreifen zu können.

### Bezugszeichenliste

- 1: Rahmen
- 2: Hohlraum
- 3: obere Öffnung des Hohlraums
- 4: balliger Randabschnitt des Rahmens
- 5: Strukturierungen
- 6: Aufnahmeöffnung
- 7: umlaufender Randbereich des Rahmens
- 8: Füllung
- 9: Kanten des Rahmens
- 10: Seitenfläche des Rahmens
- 11: untere Öffnung des Hohlraums

## Patentansprüche

1. Wirbelsäulenimplantat bestehend aus einem Rahmen (1) aus kortikalem Knochenmaterial, der mindestens einen durchgehenden Hohlraum (2) bildet, der mindestens zwei gegenüberliegende Öffnungen (3, 11) aufweist und zumindest teilweise mit spongiösem und/oder osteoinduktivem Knochenmaterial (8) gefüllt ist, wobei der Rahmen (1) im wesentlichen quaderförmig ist, wobei an dem Rahmen (1) Strukturierungen (5) vorgesehen sind, um das Wirbelsäulenimplantat zwischen benachbarten Wirbeln zu fixieren, **dadurch gekennzeichnet, daß** der Rahmen zumindest auf einer Seite ballige Randabschnitte (4) aufweist, und daß der Rahmen ( 1 ) mindestens eine Aufnahmeöffnung (6) für ein Applikationswerkzeug aufweist.

2. Wirbelsäulenimplantat nach Anspruch 1
**dadurch gekennzeichnet,**
**daß** das Material des Rahmens (1) aus konserviertem und sterilem Knochenmaterial humanen oder tierischen Ursprungs, insbesondere aus konserviertem und sterilem bovinem Knochenmaterial besteht.

3. Wirbelsäulenimplantat nach einem der vorangehenden Ansprüche
**dadurch gekennzeichnet,**
**daß** der im wesentlichen quaderförmige Rahmen ( 1 ) abgerundete Kanten (9) aufweist.

4. Wirbelsäulenimplantat nach einem der vorangehenden Ansprüche
**dadurch gekennzeichnet,**
**daß** der Rahmen ( 1 ) umlaufende Randbereiche (7) aufweist, welche jeweils die Öffnung (3, 11) des durchgehenden Hohlraums (2) umgeben.

5. Wirbelsäulenimplantat nach einem der vorangehenden Ansprüche
**dadurch gekennzeichnet,**
**daß** der Rahmen (1) auf zwei gegenüberliegenden Seiten jeweils ballige Randabschnitte (4) aufweist.

6. Wirbelsäulenimplantat nach einem der vorangehenden Ansprüche
**dadurch gekennzeichnet,**
**daß** die Füllung (8) zumindest teilweise über den Randbereich (7) des Rahmens (1) vorzugsweise um 0,5 mm übersteht.

7. Wirbelsäulenimplantat nach einem der vorangehenden Ansprüche
**dadurch gekennzeichnet,**
**daß** die Randabschnitte (4) auf zwei gegenüberliegenden Seiten des Rahmens (1) Strukturierungen (5) aufweisen, die in Form von Kerbungen, Riffelungen oder Zinnen ausgebildet sind.

8. Wirbelsäulenimplantat nach Anspruch 7,
**dadurch gekennzeichnet,**
**daß** die Füllung (8) zumindest teilweise über den Randbereich (7) vorzugsweise bis zur Höhe der freien Enden der Strukturierungen (5) übersteht.

9. Wirbelsäulenimplantat nach einem der vorangehenden Ansprüche
**dadurch gekennzeichnet,**
**daß** das Implantat in seiner Größe an den nach Ausräumung des Wirbelzwischenkörpers vorhandenen Wirbelzwischenraum zwischen benachbarten Wirbeln angepaßt ist.

10. Wirbelsäulenimplantat nach einem der vorangehenden Ansprüche
**dadurch gekennzeichnet,**
**daß** die Aufnahmeöffnung (6) auf einer oder mehreren Seiten (10) des Rahmens (1) als Bohrloch mit einem Gewinde von vorzugsweise 3 mm Tiefe ausgeführt ist.

11. Wirbelsäulenimplantat nach einem der vorangehenden Ansprüche
**dadurch gekennzeichnet,**
**daß** das spongiöse Knochenmaterial durch Lösungsmitteldehydratisieren von Knochenmaterial mittels eines organischen, mit Wasser mischbaren Lösungsmittels, z.B. mittels Methanol, Äthanol, Propanol, Isopropanol, Aceton, Methyl-Äthylketon oder Gemischen dieser Lösungsmittel, erzeugt ist.

12. Wirbelsäulenimplantat nach einem der vorangehenden Ansprüche
**dadurch gekennzeichnet,**
**daß** das spongiöse Knochenmaterial durch Lösungsmitteldehydratisieren von Knochenmaterial mit anschließender terminaler Sterilisation, insbesondere durch Bestrahlung mit Gamma-Strahlen erzeugt ist.

13. Wirbelsäulenimplantat nach einem der vorangehenden Ansprüche
**dadurch gekennzeichnet,**
**daß** das spongiöse Knochenmaterial durch aseptische Prozessierung von Knochenmaterial ohne terminale Sterilisation erzeugt ist.

## Claims

1. Spinal column implant consisting of a frame (1) of cortical bone matter which forms at least one through-going cavity (2) which has at least two oppositely disposed openings (3, 11) and is at least partly filled with spongeous and/or osteoinductive bone matter (8), with the frame (1) substantially having the shape of a right parallelepiped, with structures (5) being provided at the frame (1) in order to fix the spinal column implant between adjacent vertebrae, **characterized in that** the frame has convex edge sections at at least one side; and **in that** the frame (1) has at least one reception opening (6) for an application tool.

2. Spinal column implant in accordance with claim 1, **characterized in that** the material of the frame (1) consists of preserved and sterile bone matter of human or animal origin, in particular of preserved and sterile bovine bone matter.

3. Spinal column implant in accordance with any one of the preceding claims, **characterized in that** the frame (1), which substantially has the shape of a right parallelepiped, has rounded edges (9) .

4. Spinal column implant in accordance with any one of the preceding claims, **characterized in that** the frame (1) has peripheral edge regions (7) which respectively surround the opening (3, 11) of the through-going cavity (2).

5. Spinal column implant in accordance with any one of the preceding claims, **characterized in that** the frame (1) has respective convex edge sections (4) at two oppositely disposed sides.

6. Spinal column implant in accordance with any one of the preceding claims, **characterized in that** the filling (8) projects at least partly, preferably by 0.5 mm, beyond the edge region (7) of the frame (1).

7. Spinal column implant in accordance with any one of the preceding claims, **characterized in that** the edge sections (4) have structures (5) on two oppositely disposed sides of the frame (1) which are made in the form of notches, corrugations or pinnacles.

8. Spinal column implant in accordance with claim 7, **characterized in that** the filling (8) projects at least partly beyond the edge region (7) preferably up to the level of the free ends of the structures (5).

9. Spinal column implant in accordance with any one of the preceding claims, **characterized in that** the implant is matched in its size to the spinal column space between adjacent vertebrae present after the clearing out of the spinal column intermediate body.

10. Spinal column implant in accordance with any one of the preceding claims, **characterized in that** the reception opening (6) is made on one or more sides (10) of the frame (1) as a bore hole with a thread having a depth of preferably 3 mm.

11. Spinal column implant in accordance with any one of the preceding claims, **characterized in that** the spongeous bone matter is produced through solvent dehydrating of bone matter by means of an organic solvent which is miscible with water, e.g. by means of methanol, ethanol, propanol, isopropanol, acetone, methyl ethyl ketone or mixtures of these solvents.

12. Spinal column implant in accordance with any one of the preceding claims, **characterized in that** the spongeous bone matter is produced through solvent dehydrating of bone matter with subsequent terminal sterilization, in particular through irradiation with gamma rays.

13. Spinal column implant in accordance with any one of the preceding claims, **characterized in that** the spongeous bone matter is produced through aseptic processing of bone matter without terminal sterilization.

## Revendications

1. Implant vertébral, consistant en un cadre (1) fait en un tissu osseux cortical et formant au moins une cavité traversante (2), le cadre présentant au moins deux ouvertures (3, 11) opposées et étant rempli, au moins en partie, par un tissu osseux (8) spongieux et/ou ostéogène, dans lequel le cadre (1) est sensiblement de forme rectangulaire, dans lequel des structures façonnées (5) sont prévues sur le cadre (1) afin de fixer l'implant vertébral entre des vertèbres voisines,
**caractérisé en ce que** le cadre présente, au moins sur un côté, des parties de bord (4) bombées, et **en ce que** le cadre (1) présente au moins une ouverture de réception (6) pour un outil d'application.

2. Implant vertébral selon la revendication 1,
**caractérisé en ce que** la matière du cadre (1) consiste en un tissu osseux préservé et stérile, d'origine humaine ou animale, notamment en un tissu osseux bovin, préservé et stérile.

3. Implant vertébral selon l'une des revendications précédentes,
**caractérisé en ce que** le cadre (1) de forme sensiblement rectangulaire présente des arêtes (9) arrondies.

4. Implant vertébral selon l'une des revendications précédentes,
**caractérisé en ce que** le cadre (1) présente, tout autour, des zones de bord (7) qui entourent chaque fois l'ouverture (3, 11) de la cavité continue (2).

5. Implant vertébral selon l'une des revendications précédentes,
**caractérisé en ce que** le cadre (1) présente, sur deux côtés opposés, chaque fois des parties de bord (4) bombées.

6. Implant vertébral selon l'une des revendications précédentes,
**caractérisé en ce que** le remplissage (8) dépasse, au moins en partie, de la zone de bord (7) du cadre (1) et, de préférence de 0,5 mm.

7. Implant vertébral selon l'une des revendications précédentes,
**caractérisé en ce que** les parties de bord (4), situées sur deux côtés opposés du cadre (1), présentent des structures façonnées (5), qui sont réalisées en forme d'entailles, de cannelures ou de créneaux.

8. Implant vertébral selon la revendication 7,
**caractérisé en ce que** le remplissage (8) dépasse, au moins en partie, de la zone de bord (7), de préférence jusqu'à la hauteur des extrémités libres des structures façonnées (5).

9. Implant vertébral selon l'une des revendications précédentes,
**caractérisé en ce que** l'implant est adapté, quant à sa taille, à l'espace intervertébral, disponible entre des vertèbres voisines après l'enlèvement du corps intervertébral.

10. Implant vertébral selon l'une des revendications précédentes,
**caractérisé en ce que** l'ouverture de réception (6) est réalisée, sur un seul ou sur plusieurs côtés (10) du cadre (1), en tant qu'alésage avec un taraudage, de préférence, de 3 mm de profondeur.

11. Implant vertébral selon l'une des revendications précédentes,
**caractérisé en ce que** le tissu osseux spongieux est obtenu par déshydratation par solvant de tissu osseux à l'aide d'un solvant organique, propre à être mélangé avec de l'eau, par exemple à l'aide de méthanol, d'éthanol, de propanol, d'isopropanol, d'acétone, de méthyléthylcétone ou de mélanges de ces solvants.

12. Implant vertébral selon l'une des revendications précédentes,
**caractérisé en ce que** le tissu osseux spongieux est obtenu par déshydratation par solvant de tissu osseux, avec ensuite une stérilisation finale, notamment par exposition à des rayons gamma.

13. Implant vertébral selon l'une des revendications précédentes,
**caractérisé en ce que** le tissu osseux spongieux est obtenu par un traitement aseptique du tissu osseux sans stérilisation finale.
